# EUROPÄISCHE PATENTANMELDUNG

(11) **EP 0 602 362 A1**
(43) Veröffentlichungstag der Anmeldung: **22.06.1994**
(21) Anmeldenummer: 93117648.1
(22) Anmeldetag: 30.10.1993
(51) Int. Cl.: A61N 5/06, F21V 9/04

(54) **Bestrahlungsanordnung**

(30) Priorität: 11.11.1992 DE 4237967; 26.11.1992 DE 4239675
(71) Anmelder: LUXMEDICA GESELLSCHAFT FÜR LICHTTECHNIK mbH, D-44879 Bochum (DE)
(72) Erfinder:
(74) Vertreter: Herrmann-Trentepohl, Werner, Dipl.-Ing.

(57) **Zusammenfassung**

Bei einer Bestrahlungsanordnung, bei der in dem aus einer Lampe (3) mit Reflektor (4) austretenden Licht mindestens ein Scheibenfilter (7) angeordnet ist, das zum Ausblenden von Lichtwellen dient, deren Wellenlängen oberhalb einer Grenzwellenlänge liegt, während das unterhalb der Grenzwellenlänge liegende Licht das Filter durchdringt, kennzeichnet sich die Erfindung dadurch, daß das Filter (7) wenigstens eine Filterscheibe (5, 6) aufweist, die derart geneigt angeordnet ist, daß sie im wesentlichen die auszublendenden Lichtwellen reflektiert und sie seitlich aus dem Strahlengang nach außen ablenkt, während sie das unterhalb der Grenzwellenlänge liegende Licht durchläßt.

## Beschreibung

Die Erfindung betrifft eine Bestrahlungsanordnung gemäß dem Oberbegriff des Anspruches 1.

Obwohl die Erfindung auch auf andere Zweckverwendungen anwendbar ist, bezieht sie sich insbesondere auf Bestrahlungseinrichtungen für die medizinische Anwendung in der Lichttherapie unter Verwendung von Lichtquellen, die inkohärentes Licht aussenden. Erfindungsgemäß ist als Lichtquelle vorzugsweise eine Metalldampflampe vorgesehen, welche erforderliche Strahlungsdichte auf der zu behandelnden Körperstelle des Patienten herbeiführt. Solche Lichtquellen senden außer dem für die Behandlung erwünschten Lichtspektrum auch Lichtwellen aus, deren Wellenlänge oberhalb einer Grenzwellenlänge liegt, die das verwendbare Lichtspektrum begrenzt, das in der Regel von kurzwelligem Rotlicht gebildet wird. Da die langwelligen Infrarotstrahlungen zu Verbrennungen, insbesondere auf der Haut eines mit Licht therapierten Patienten führen können, weil sie nicht in das darunterliegende Gewebe eindringen, werden erfindungsgemäß diese Lichtspektren ausgeblendet, so daß nur das kurzwellige Infrarotlicht durchdringt und in das tiefer liegende Gewebe eindringen kann, wo es seine heilende Wirkung entfaltet.

Zum Ausblenden der unerwünschten Lichtspektren verwendet die Erfindung ein Filter mit einer oder mehreren Filterscheiben, die aus optischem Glas bestehen. Beispielsweise werden diese Filterscheiben so ausgewählt, daß die Grenzwellenlänge unterhalb 700 nm liegt, wobei weitere Grenzwellenlängen zwischen 700 bis 900 nm und 1100 bis 1300 nm liegen.

Derartige Bestrahlungsanordnungen sind bekannt und werden in der Regel in einem Gehäuse untergebracht, aus dem das gefilterte Licht austritt, das im wesentlichen in dem gewählten kurzwelligen Bereich liegt (DE-GM 81 20 029.3). Hierbei werden für die Ausblendung vergleichsweise einfache Glasscheiben verwendet, die hintereinander im Strahlengang in parallelen Ebenen angeordnet sind, welche dessen optische Achse im wesentlichen rechtwinklig schneiden. Jede Filterscheibe absorbiert ein bestimmtes Lichtwellenspektrum, so daß am Ende des Strahlenganges nur das Licht mit den gewünschten Wellenlängen aus dem Gehäuse austritt. Bei derartigen Geräten ergibt sich jedoch die Schwierigkeit, daß bei Lichtquellen mit hoher Leistung die Absorption der unerwünschten Lichtspektren die Filterscheiben aufheizt. Die zerstörungsfreie Aufnahme der dabei auftretenden Temperaturen setzt eine ständige, großflächige Kühlung der Gläser des Filters voraus. Das führt im Ergebnis zur Begrenzung der maximal möglichen Lichtleistung und der zur Therapie verfügbaren Strahlungsdichte.

Die Erfindung geht demgegenüber einen anderen Weg, dessen Grundgedanke im Anspruch 1 wiedergegeben ist. Weitere Merkmale der Erfindung sind Gegenstand der Unteransprüche.

Gemäß der Erfindung besteht das Filter aus wenigstens einer Filterscheibe, die das auszublendende Lichtspektrum nicht mehr absorbiert, sondern in Folge ihrer geneigten Anordnung seitlich aus dem Strahlengang reflektiert und dabei Lichtwellen des gewünschten Spektrums durchläßt. Dadurch wird erreicht, daß sich die Aufheizung des Filters auch bei hohen Lichtleistungen in Grenzen hält, so daß grundsätzlich die gewünschte Strahlungsdichte nicht durch das Filter eingeschränkt ist.

Vorzugsweise wird die Erfindung mit den Merkmalen des Anspruches 2 verwirklicht. Das geschieht auf besonders einfache Weise dadurch, daß man jede Filterscheibe auf zwei Segmente aufteilt, die auf den Schenkeln eines spitzen Winkels liegen, dessen Scheitel etwa in der Mitte des Strahlengangs angeordnet ist, wodurch die beiden Segmente jeweils das auszublendende Lichtspektrum annähernd zur Hälfte übernehmen. Die ausgeblendeten Lichtwellen treten dadurch an beiden Seiten des Strahlengangs seitlich aus.

Es ist ferner zweckmäßig, die Erfindung mit den Merkmalen des Anspruches 3 zu verwirklichen. Hierbei wird das inkohärente Licht des gewünschten Spektrums in einer Sammellinse zusammengeführt, wodurch eine erhebliche Strahlungsdichte auf einem begrenzten Bezirk erzielt wird.

Die erfindungsgemäße Strahlungsanordnung eignet sich auch zur Unterbringung in einem Gehäuse. Hierbei treffen die seitlich aus dem Strahlengang reflektierten ausgeblendeten Lichtwellen auf das Gehäuse auf und werden von den getroffenen Gehäuseteilen im wesentlichen absorbiert. Dies geschieht auf einer verhältnismäßig großen Gehäusefläche, wodurch man die Möglichkeit hat, durch Belüftung des Gehäuses dessen maximale Aufheizung zu begrenzen.

Die Erfindung kann außerdem mit Filtern verwirklicht werden, die aus mehreren hintereinandergeschalteten Filterscheiben bestehen, welche sich voneinander durch unterschiedliche Neigungswinkel entsprechend den jeweils auszufilternden Lichtspektren unterscheiden. Diese aus Segmenten zusammengesetzten Filterscheiben sind so hintereinander angeordnet, daß die von ihnen reflektierten Strahlen an der jeweils benachbarten Filterscheibe vorbeireflektiert werden, wodurch deren Aufheizung verhindert wird.

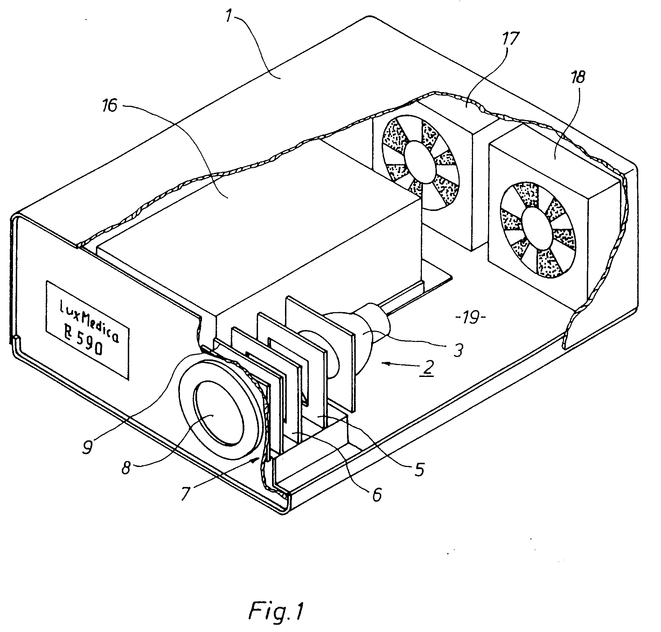

Die Einzelheiten, weiteren Merkmale und andere Vorteile der Erfindung ergeben sich aus der nachfolgenden Beschreibung eines Ausführungsbeispiels anhand der Figuren in der Zeichnung; es zeigen
- Fig. 1: in perspektivischer Darstellung ein Bestrahlungsgerät mit der erfindungsgemäßen Bestrahlungsanordnung in perspektivischer Darstellung,
- Fig. 2: den Gegenstand der Fig. 1 im Längsschnitt durch die Bestrahlungsanordnung und
- Fig. 3: schematisch den Strahlengang einer abgeänderten Ausführungsform, die eine Filterscheibe als Filter verwendet.

In einem Metallgehäuse 1 sind sämtliche funktionsnotwendigen Teile einer Bestrahlungsanordnung 2 untergebracht. Die Bestrahlungsanordnung besteht aus einer Metalldampflampe 3 mit einem Reflektor 4 und zwei Interferenzfilterscheiben 5 und 6, die das allgemein mit 7 bezeichnete Filter bilden. Das gefilterte Licht tritt aus einer Sammellinse 8 aus und wird auf die zu behandelnde Stelle des zu therapierenden Patienten gerichtet. Hinter den Infrarotinterferenzfilterscheiben 5 und 6 befindet sich eine Absorptionsfilterscheibe 9, welche das nicht ausgefilterte kurzwellige Licht absorbiert.

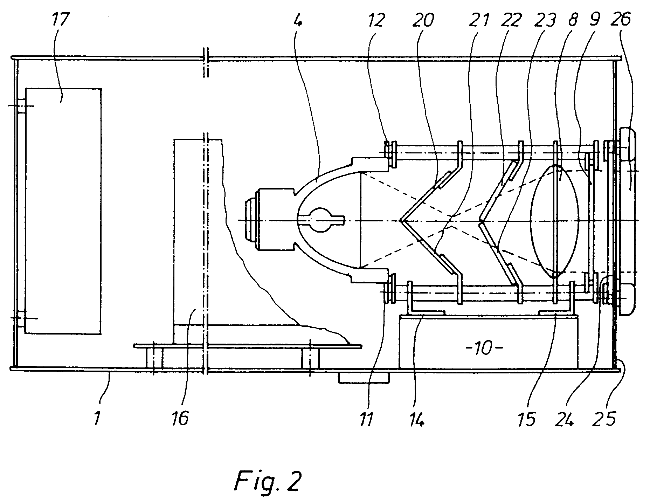

Die beschriebenen Teile sind auf einer Bühne 10 innerhalb des Gehäuses 1 aufgebaut (Fig. 2). Dazu dient ein Gestängegerüst 11, 12, welches die geforderten Abstände der beschriebenen Bauteile des Filters gewährleistet und zur Befestigung mit Winkeln 14, 15 auf der Bühne 10 dient.

Innerhalb des Gehäuses befindet sich in einem weiteren Gehäuse 16 die Steuerungselektronik, mit der auch zwei Ventilatoren 17, 18 gesteuert werden können. Diese Ventilatoren dienen zur Belüftung des Innenraumes 19 des Gehäuses 1 und zur Kühlung der Gehäusewände.

Wie sich aus der Darstellung der Fig. 2 ergibt, besteht jede Filterscheibe 5, 6 aus zwei Segmenten 20, 21 bzw. 22, 23. Diese Segmente sind jeweils so angeordnet, daß die auszublendenden Lichtanteile aus dem Strahlengang reflektiert werden, während die in dem gewünschten Grenzspektrum liegenden, langwelligen Infrarotstrahlen von den Segmenten durchgelassen werden. Diese Lichtwellen treffen auf die Linse 8, wo sie weitgehend parallel gerichtet werden und über die Filterscheibe 9 durch die Öffnung 24 in der Stirnseite 25 des Gehäuses austreten, wo sie zunächst einen Ring 26 durchlaufen, der zum Schutz der Öffnung 24 und der dahinterliegenden Baugruppen dient. Das ausgeblendete Licht beider Filter 5, 6 trifft auf das Gehäuse, wobei durch die Winkelstellung der Segmente 22, 23 die von dem Filter reflektierten Strahlen an den Segmenten 20, 21 vorbeigelenkt werden.

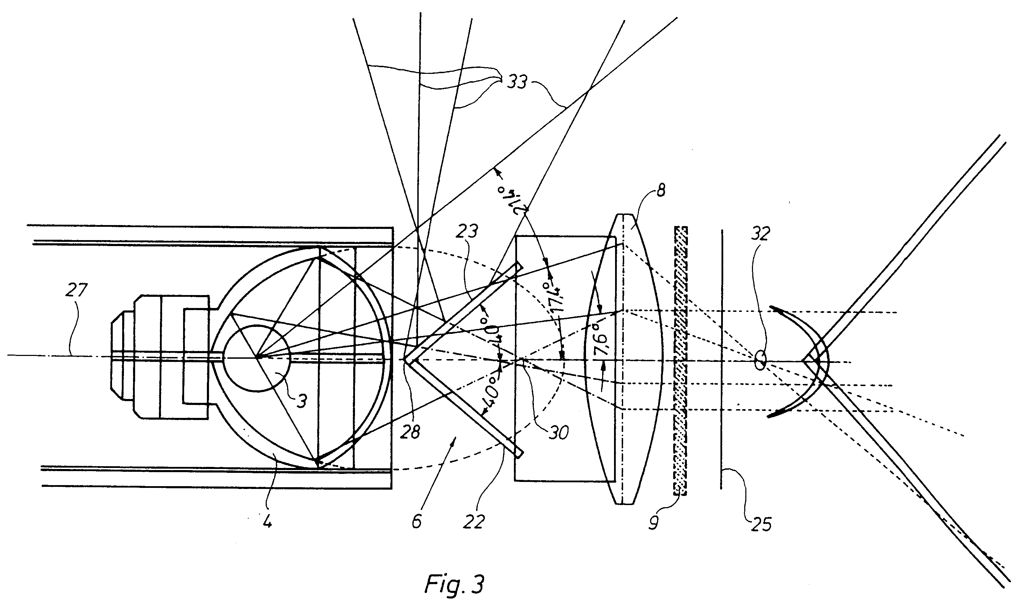

Die Fig. 3 zeigt eine Ausführungsform, welche nur eine Filterscheibe 6 verwendet, deren Segmente 22, 23 jeweils mit der optischen Achse 27 einen Winkel von 40° einschließen. Die beiden Segmente 22, 23 liegen auf den Schenkeln eines Winkels von 80°, dessen Scheitel 28 auf der optischen Achse 27 angeordnet ist. Die Metalldampflampe 3 liegt in dem ellipsoiden Reflektor 4, dessen Brennpunkt bei 30 auf der optischen Achse 27 liegt. In den Strahlengang ist die konvexe Linse 8 eingeschaltet, welche die aus dem Reflektor austretenden direkt von der Lichtquelle ausgehenden gefilterten Strahlen in ihrem Bildpunkt 32 zusammenführt. Der Brennpunkt ist durch die Ebene 25 gegeben. Der Punkt 32 liegt auf der Abbildungsebene der Lampe 3. Die bei 33 beispielsweise dargestellten, reflektierten Strahlen zeigen, daß durch die gewählte Winkelstellung der Filtersegmente das ausgeblendete Licht nach außen aus dem Strahlengang auf das Gehäuse gerichtet ist.

## Patentansprüche

1. Bestrahlungsanordnung, bei der in dem aus einer Lampe (3) mit Reflektor (4) austretenden Licht mindestens ein Scheibenfilter (7) angeordnet ist, das zum Ausblenden von Lichtwellen dient, deren Wellenlängen oberhalb einer Grenzwellenlänge liegt, während das unterhalb der Grenzwellenlänge liegende Licht das Filter durchdringt, dadurch gekennzeichnet, daß das Filter (7) wenigstens eine Filterscheibe (5, 6) aufweist, die derart geneigt angeordnet ist, daß sie im wesentlichen die auszublendenden Lichtwellen reflektiert und sie seitlich aus dem Strahlengang nach außen ablenkt, während sie das unterhalb der Grenzwellenlänge liegende Licht durchläßt.

2. Bestrahlungsanordnung nach Anspruch 1, dadurch gekennzeichnet, daß die Filterscheibe (5, 6) aus zwei Segmenten (20, 21; 22, 23) aufgebaut ist, die einen spitzen Winkels einschließen, dessen Scheitel (28) etwa in der Mitte des Strahlengangs derart angeordnet ist, daß die optische Achse (27) die Winkelhalbierende bildet.

3. Bestrahlungsanordnung nach den Ansprüchen 1 oder 2, dadurch gekennzeichnet, daß in den aus dem Filter (7) austretenden Strahlengang eine bikonvexe Linse (8) eingeschaltet ist, wobei das zu bestrahlende Objekt im wesentlichen im Brennpunkt (25) der Linse (8) angeordnet ist.

4. Bestrahlungsanordnung nach einem der Ansprüche 1 bis 3, dadurch gekennzeichnet, daß der Linse (8) ein Absorptionsfilter (9) nachgeschaltet ist.

5. Bestrahlungsanordnung nach einem der Ansprüche 1 bis 4, dadurch gekennzeichnet, daß sie in einem Gehäuse (1) untergebracht ist, auf das die reflektierten Strahlen auftreffen und von dem die reflektierten Strahlen im wesentlichen absorbiert werden.

6. Bestrahlungsanordnung nach einem der Ansprüche 1 bis 5, dadurch gekennzeichnet, daß der Innenraum (19) des Gehäuses mit einem oder mehreren Lüfter (17, 18) gekühlt ist.

7. Bestrahlungsanordnung nach einem der Ansprüche 1 bis 6, dadurch gekennzeichnet, daß mehrere Filterscheiben (5, 6) hintereinandergeschaltet und mit ihren jeweiligen, voneinander abweichenden Neigungswinkeln derart angeordnet sind, daß sie Teilspektren der auszublenden Wellenlängen seitlich aus dem Strahlengang an der benachbarten Filterscheibe (5) vorbeireflektieren.

8. Bestrahlungsanordnung nach einem der Ansprüche 1 bis 7, dadurch gekennzeichnet, daß das gefilterte Licht aus einer Gehäuseöffnung (24) austritt, hinter der im Gehäuse die Bestrahlungsanordnung befestigt ist, wobei auf der die Öffnung (24) aufweisenden Stirnseite (25) des Gehäuses (1) ein Schutzring (26) angebracht ist.
